(19) Europäisches Patentamt
European Patent Office
Office européen des brevets

(11) Publication number: **0 237 899 B1**

(12) # EUROPEAN PATENT SPECIFICATION

(45) Date of publication of patent specification: **11.09.91**

(51) Int. Cl.5: **C07D 265/36**, C07D 241/44, C07C 205/12, //C07D401/04, C07D413/04,A01N43/84, A01N43/60

(21) Application number: **87103324.7**

(22) Date of filing: **09.03.87**

The file contains technical information submitted after the application was filed and not included in this specification

(54) **Production of fluoroaniline derivatives.**

(30) Priority: **10.03.86 JP 52300/86**
**12.03.86 JP 53921/86**
**14.11.86 JP 272570/86**
**14.11.86 JP 272571/86**
**30.01.87 JP 21223/87**

(43) Date of publication of application:
**23.09.87 Bulletin 87/39**

(45) Publication of the grant of the patent:
**11.09.91 Bulletin 91/37**

(84) Designated Contracting States:
**CH DE FR GB IT LI NL**

(56) References cited:
**EP-A- 0 170 191**

(73) Proprietor: **SUMITOMO CHEMICAL COMPANY, LIMITED**
**Kitahama 4-chome 5-33**
**Chuo-ku Osaka 541(JP)**

(72) Inventor: **Takemoto, Ichiki**
**10-1-124, Sonehigashi-machi 2-chome**
**Toyonaka-shi Osaka-fu(JP)**

(74) Representative: **Vossius & Partner**
**Siebertstrasse 4 P.O. Box 86 07 67**
**W-8000 München 86(DE)**

EP 0 237 899 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the production of fluoroaniline derivatives. More particularly, it relates to the production of fluoroaniline derivatives of the formula:

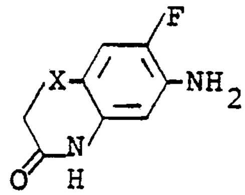

(I)

wherein X is an oxygen atom or an imino group useful as intermediates in the synthesis of agrochemicals.

In the present application, the term "lower" is intended to mean any group having not more than 8 carbon atoms, preferably not more than 5 carbon atoms.

It is known that some tetrahydrophthalimide derivatives are useful as herbicides. For instance, 2-(7-fluoro-4-propargyl-2H-1,4-benzoxadin-3-(4H)-on-6-yl)-4,5,6,7-tetrahydroisoindole-1,3-dione is known to exert a strong herbicidal potency. However, this compound is usually produced from 2-nitro-5-fluorophenoxyacetic acid, which can be manufactured only through various steps with troublesome operations. Thus, the conventional process is not satisfactory from the industrial viewpoint.

EP-A-0170191 discloses the preparation of tetrahydrophthalimides. The preparation process involves a four step procedure with a m-halophenol as starting material for the preparation of an intermediate corresponding to the fluoroaniline derivative of the present invention.

In order to provide an advantageous process for industrial production of said tetrahydrophthalimide derivatives, an extensive study has been carried out and, as a result, it has now been found that the use of the fluoroaniline derivatives (I) as the intermediates can afford the objective tetrahydrophthalimide derivatives with great industrial advantages.

The fluoroaniline derivatives (I) can be produced by

(a) reacting 1,5-dichloro-2,4-dinitrobenzene with a glycol derivative of the formula:

$Y\text{-}CH_2\text{-}XH$ (III)

wherein X is as defined above and Y is a cyano group, a carboxy group or a lower alkoxycarbonyl group to produce a chlorodinitrobenzene derivative of the formula

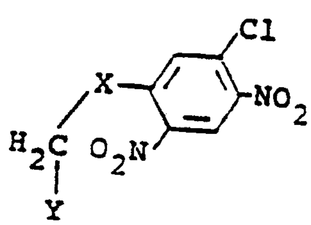

(IV)

wherein X and Y are each as defined above;

(b) reacting the chlorodinitrobenzene derivative (IV) with a metal fluoride to produce a fluorodinitrobenzene derivative of the formula:

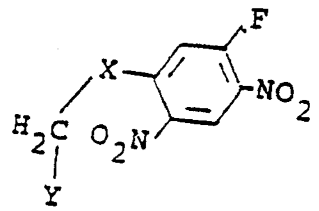

(II)

wherein X and Y are as defined above; and

(c) subjecting the fluorodinitrobenzene to reductive cyclization; or by

(a) reacting 1,5-difluoro-2,4-dinitrobenzene with a glycol derivative of the formula:

$Y-CH_2-XH$ (III)

wherein X is as defined above and Y is a cyano group, a carboxy group or a lower alkoxycarbonyl group to produce a fluorodinitrobenzene derivative of the formula

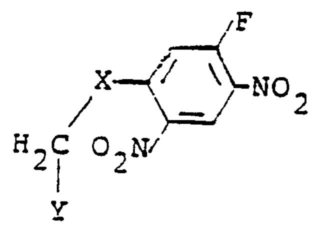

(II)

wherein X and Y are each as defined above, and

(b) subjecting the fluorodinitrobenzene to reductive cyclization.

Examples of the fluorodinitrobenzene derivative (II) are 2,4-dinitro-5-fluorophenoxyacetic acid, methyl 2,4-dinitro-5-fluorophenoxyacetate, ethyl 2,4-dinitro-5-fluorophenoxyacetate, 2,4-dinitro-5-fluorophenoxyacetonitrile, N-(2,4-dinitro-5-fluorophenyl)glycine ethyl ester, N-(2,4-dinitro-5-fluorophenyl)aminoacetonitrile, etc.

The reductive cyclization may be achieved by any appropriate procedure such as catalytic reduction or chemical reduction.

The catalytic reduction may be effected in the presence of a catalyst such as platinum dioxide, palladiumcarbon or Raney-nickel under a hydrogen atmosphere. The amount of the catalyst may be from catalytic to excessive, preferably from catalytic to 60 % by weight, to the fluorodinitrobenzene derivative (II). It is normally carried out in an inert solvent such as an organic solvent (e.g. methanol, ethanol, isopropanol, ethyl acetate, tetrahydrofuran, toluene, xylene), water or their mixtures. The reaction is effected usually at a temperature of room temperature to 150 °C under an ordinary or elevated pressure for a period of 1 to 10 hours.

The chemical reduction is usually performed in the presence of iron powder and an acid. The amount of iron powder to be used may be not less than 5 mole, preferably from 6 to 10 mole, to one mole of the fluorodinitrobenzene derivative (II). As the acid, there may be employed an inorganic acid (e.g. hydrochloric acid, sulfuric acid), an organic acid (e.g. acetic acid), etc. It is usually carried out in an inert solvent, of which examples are water, an organic solvent (e.g. acetic acid, methanol, ethanol, isopropanol, tetrahydrofuran) and their mixtures. In general, the reaction is performed at a temperature of room temperature to 200 °C, preferably of 80 to 150 °C, under an ordinary pressure for a period of 1 to 20 hours.

Simultaneously with the reduction, the cyclization takes place to give the fluoroaniline derivative (I) in an excellent yield.

The fluorodinitrobenzene derivative (II) can be produced, for instance, by reacting 1,5-difluoro-2,4-dinitrobenzene with a glycol derivative of the formula:

$Y-CH_2-XH$ (III)

wherein X and Y are each as defined above in an amount of 1 to 1.5 equivalents to said compound, usually in the presence of a basic catalyst in an inert solvent.

As the glycol derivative (III), there may be employed glycollic acid, lower alkyl glycolate (e.g. methyl glycolate, ethyl glycolate, propyl glycolate, butyl glycolate), glycine lower alkyl esters (e.g. glycine methyl ester, glycine ethyl ester, glycine n-butyl ester), glycolonitrile, aminoacetonitrile, etc. The amount of the glycol derivative (III) to be used is usually from 1 to 1.5 equivalents to said 1,5-difluoro-2,4-dinitrobenzene. Examples of the basic catalyst are sodium hydroxide, potassium hydroxide, potassium carbonate, sodium hydrogen carbonate, dipotassium hydrogen phosphate, tripotassium phosphate, pyridine, triethylamine, N,N-dimethylaniline, etc. The amount of the basic catalyst may be from a catalytic one to 4 equivalents, preferably from 1 to 3 equivalents, to said 1,5-difluoro-2,4-dinitrobenzene. Examples of the inert solvent are an organic solvent (e.g. toluene, xylene, acetone, tetrahydrofuran, ethyl acetate, methylene chloride, chloroform, chlorobenzene, acetonitrile, N,N-dimethylformamide, dimethylsulfoxide), water and their mixtures. In general, the reaction is carried out at a temperature of -50 to 200 °C, preferably of -20 to 150 °C, for a period of 1 to 10 hours.

The fluorodinitrobenzene derivative (II) can be also produced by reacting a chlorodinitrobenzene derivative of the formula:

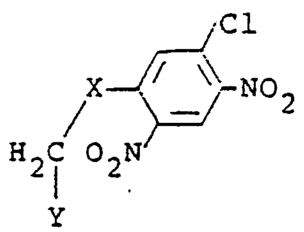

(IV)

where in X and Y are each as defined above with a metal fluoride, usually in an inert solvent.

As the metal fluoride, there may be used CsF, KF, NaF, $CaF_2$ or the like. Among them, KF is particularly preferred. The amount of the metal fluoride is usually from 1 to 5 equivalents, preferably from 1 to 3 equivalents, to the chlorodinitrobenzene derivative (IV). The presence of a phase transfer catalyst such as 18-crown-6 and TDA-1 in an amount of a catalytic one to 2 equivalents, preferably of 0.001 to 0.5 equivalent, to the chlorodinitrobenzene derivative (IV) in the reaction system is particularly favorable. Examples of the inert solvent are organic solvents (e.g. toluene, xylene, acetone, tetrahydrofuran, ethyl acetate, methylene chloride, chloroform, chlorobenzene, acetonitrile, N,N-dimethylformamide, dimethylsulfoxide) and their mixtures at a temperature of 0 to 200 °C, preferably of 50 to 150 °C, for a period of 1 to 10 hours.

Still, the chlorodinitrobenzene derivative (IV) may be prepared, for instance, by reacting 1,5-dichloro-2,4-dinitrobenzene with the glycol derivative (III), usually in the presence of a catalyst in an inert solvent. The glycol derivative (III) may be used in an amount of 1 to 5 equivalents, preferably of 1 to 2 equivalents, to said 1,5-dichloro-2,4-dinitrobenzene. As the catalyst, there is usually employed a basic one, of which examples are sodium hydroxide, potassium hydroxide, potassium carbonate, sodium hydrogen carbonate, dipotassium hydrogen phosphate, tripotassium phosphate, pyridine, triethylamine, N,N-dimethylaniline, etc. The amount of the basic catalyst is normally from a catalytic one to 5 equivalents, preferably from 1 to 2 equivalents to said 1,5-dichloro-2,4-dinitrobenzene. In addition to said basic catalyst, there may be used a copper catalyst (e.g. cuprous chloride, cuprous oxide, copper powder) or a phase transfer catalyst (e.g. 18-crown-6, TDA-1). Examples of the inert solvent are organic solvents (e.g. toluene, xylene, acetone, tetrahydrofuran, ethyl acetate, methylene chloride, chloroform, chlorobenzene, acetonitrile, N,N-dimethylformamide, dimethylsulfoxide), water and their mixtures. The reaction is generally performed at a temperature of -50 to 200 °C, preferably of -20 to 150 °C, for a period of 1 to 30 hours.

As explained hereinabove, this invention can afford the fluoroaniline derivatives (I) in excellent yields. These fluoroaniline derivatives (I) can be readily converted into the corresponding tetrahydrophthalimide derivatives which are per se useful as agrochemicals. Thus, this invention provides industrially advantageous processes for the production of the fluoroaniline derivatives (I) as well as the tetrahydrophthalimide derivatives.

The present invention will now be explained further in detail by the following Examples wherein % is by weight unless otherwise indicated.

Example 1

Production of the chlorodinitrobenzene derivative (IV):-

(1) A solution of 1,5-dichloro-2,4-dinitrobenzene (5.0 g), glycolonitrile (1.81 g) and anhydrous potassium carbonate (2.19 g) in dimethylformamide (20 g) was stirred at 25 to 30 °C for 24 hours. After completion of the reaction, the reaction mixture was diluted with water (200 ml) and extracted with ethyl acetate (200 ml). The organic layer was washed with water, dried over magnesium sulfate and concentrated. The precipitated crystals were collected by filtration and washed with ether to give crude crystals (4.16 g), which were recrystallized from methanol to give 2,4-dinitro-5-chlorophenoxyacetonitrile (3.18 g; yield, 58.5 %). M.P., 117 - 118 °C.
Elementary analysis for $C_8H_4ClN_3O_5$:
Calcd.: C, 37.30 %; H, 1.57 %; N, 16.31 %; Cl, 13.77 %.
Found: C, 37.66 %; H, 1.66 %; N, 16.55 %; Cl 13.74 %.
NMR $\delta$ ($CDCl_3$-DMSO-$d_6$): 5.40 (2H, s), 7.89 (1H, s), 8.20 (1H, s).
IR Nujol ($cm^{-1}$): 1580 (benzene ring); 1340 (nitro).

(2) In the same manner as above but using triethylamine (3.2 g) in place of anhydrous potassium carbonate, there was obtained 2,4-dinitro-5-chlorophenoxyacetonitrile (3.02 g; yield, 55.6 %).

(3) A solution of 1,5-dichloro-2,4-dinitrobenzene (2.0 g), ethyl glycolate (1.32 g) and anhydrous potassium carbonate (0.88 g) and TDA-1 (0.27 g) in acetonitrile (10 g) was refluxed for 5 hours. After cooling, the reaction mixture was diluted with 3 % hydrochloric acid (200 ml) and extracted with ethyl acetate. The organic layer was washed with 5 % sodium carbonate solution, water and saturate sodium chloride solution in order, dried over magnesium sulfate, filtered and concentrated. The precipitated crystals were collected by filtration and recrystallized from methanol to give ethyl 2,4-dinitro-5-chlorophenoxyacetate (1.9 g; yield, 73.9 %). M.P., 129 - 130 °C.
Elementary analysis for $C_{10}H_9ClN_2O_7$:
Calcd.: C, 39.42 %; H, 2.98 %; N, 9.20 %; Cl, 11.64 %.
Found: C, 39.27 %; H, 2.99 %; N, 9.15 %; Cl, 11.54 %.
NMR $\delta$ ($CDCl_3$-DMSO-$d_6$): 1.30 (3H, t, J = 8Hz), 4.28 (2H, q, J = 8 Hz), 5.00 (2H, s), 7.40 (1H, s), 8.68 (1H, s).
IR Nujol ($cm^{-1}$): 1720 (ester); 1580 (benzene ring); 1340 (nitro).

Example 2

Production of the fluorodinitrobenzene derivative (II):-

(1) A suspension of 2,4-dinitro-5-chlorophenoxyacetonitrile (5.0 g) and potassium fluoride (1.7 g) in dimethylsulfoxide (25 g) was stirred at 83 to 85 °C for 1 hour. The reaction mixture was poured into ice-water (300 ml) and extracted with ethyl acetate. The organic layer was washed with water, dried over magnesium sulfate and concentrated. The precipitated crystals were collected by filtration and washed with ether to give crystalline 2,4-dinitro-5-fluorophenoxyacetonitrile (4.18 g; yield, 89.3 %). M.P., 118 - 118.5 °C (recrystallized from methanol).
Elementary analysis for $C_8H_4FN_3O_5$:
Calcd.: C, 39.85 %; H, 1.67 %; N, 17.43 %.
Found: C, 39.72 %; H, 1.83 %; N, 17.38 %.
NMR $\delta$ ($CDCl_3$-DMSO-$d_6$): 8.88 (1H, d, J = 8 Hz), 7.84 (1H, d, J = 13 Hz), 5.40 (2H, s).
IR Nujol ($cm^{-1}$): 1590 (benzene ring); 1350 (nitro).

(2) A solution of 2,4-dinitro-5-chlorophenoxyacetonitrile (2.0 g), potassium fluoride (0.54 g) and 18-crown-6 (0.21 g) in acetonitrile (20 ml) was heated under reflux for 2 hours. The reaction mixture was poured into ice-water (200 ml) and extracted with ethyl acetate. Post-treatment in the same manner as above gave crystalline 2,4-dinitro-5-fluorophenoxyacetonitrile (1.7 g; yield, 90.8 %).

(3) To a suspension of 1,5-difluoro-2,4-dinitrobenzene (2.0 g) and aminoacetonitrile sulfate (2.0 g) in acetone (40 g) kept below 8 °C, a solution of sodium hydrogen carbonate (3.3 g) in water (50 g) was dropwise added, followed by stirring for 3.5 hours under ice-cooling. The reaction mixture was poured into ice-water and extracted with ethyl acetate. The organic layer was washed with water and concentrated to give N-(2,4-dinitro-5-fluorophenyl)aminoacetonitrile (2.06 g; yield, 87.5 %). M.P., 166 - 167 °C (recrystallized from methanol).
Elementary analysis for $C_8H_5FN_4O_4$:
Calcd.: C, 40.01 %; H, 2.10 %; N, 23.33 %.
Found: C, 40.01 %; H, 1.91 %; N, 23.39 %.

NMR δ ($CDCl_3$-DMSO-$d_6$): 9.10 (1H, d, J = 8 Hz), 9.1 (1H, m), 7.19 (1H, d, J = 14 Hz), 4.65 (2H, d, J = 6 Hz).
IR Nujol ($cm^{-1}$): 3300 (NH), 1610 (benzene ring), 1570 (nitro).
(4) To a solution of 1,5-difluoro-2,4-dinitrobenzene (7.4 g) and triethylamine (4.4 g) in toluene (74 g) kept at room temperature, a mixture of glycolonitrile (3.1 g) and toluene (6.2 g) was dropwise added, followed by stirring at room temperature for 2 hours. The reaction mixture was poured into 5 % hydrochloric acid and extracted with ethyl acetate. The organic layer was washed with water and concentrated to give 2,4-dinitro-5-fluorophenoxyacetonitrile (8.2 g; yield, 93.8 %). M.P., 112 - 113 °C (recrystallized from ethyl acetate-hexane).
NMR δ ($CDCl_3$-DMSO-$d_6$): 8.88 (1H, d, J = 8 Hz), 7.84 (1H, d, J = 13 Hz), 5.40 (2H, s).
IR Nujol ($cm^{-1}$): 1600 (benzene ring), 1510 and 1340 (nitro).
EI-MS m/z: 241 ($M^+$), 186, 169, 97.
(5) To a suspension of 1,5-difluoro-2,4-dinitrobenzene (3.8 g) and anhydrous potassium carbonate (3.1 g) in acetonitrile (50 ml) kept at room temperature, a solution of glycolonitrile (1.5 g) in acetonitrile (10 ml) was dropwise added, followed by stirring at room temperature for 1 hour. The reaction mixture was heated and allowed to reflux for 30 minutes (inner temperature, 83 °C). After cooling, the resultant mixture was poured into water and extracted with ethyl acetate. The organic layer was washed with water and concentrated to give crystalline 2,4-dinitro-5-fluorophenoxyacetonitrile (4.0 g; yield, 89.1 %).
(6) To a mixture of 1,5-difluoro-2,4-dinitrobenzene (2 g), acetone (20 g) and 50 % aqueous glycolonitrile solution (1.2 g), a solution of sodium hydrogen carbonate (0.9 g) in water (18 g) was dropwise added at 27 to 30 °C in 5 minutes, and stirring was continued at 27 to 41 °C for 4.5 hours. After completion of the reaction, the reaction mixture was poured into 1 % hydrochloric acid, extracted with ethyl acetate and concentrated. The precipitated crystals were collected by filtration and dried to give 2,4-dinitro-5-fluorophenoxyacetonitrile (2.0 g; yield, 83.0 %).
(7) A suspension of ethyl 2,4-dinitro-5-chlorophenoxyacetate (2.0 g) and potassium fluoride (0.57 g) in dimethylsulfoxide (10 g) was stirred at a temperature of 90 to 101 °C. After cooling, the reaction mixture was diluted with ice water (200 ml) and extracted with ethyl acetate. The organic layer was washed with water and saturated sodium chloride solution in order, dried over anhydrous magnesium sulfate and concentrated to give ethyl 2,4-dinitro-5-fluorophenoxyacetate (1.7 g; yield, 89.9 %). M.P., 59.0 - 59.5 °C (recrystallized from methanol).
Elementary analysis for $C_{10}H_9FN_2O_7$:
Calcd.: C, 41.67 %; H, 3.15 %; N, 9.72 %.
Found: C, 41.51 %; H, 3.08 %; N, 9.61 %.
NMR δ ($CDCl_3$-DMSO-$d_6$): 1.35 (3H, t, J = 8 Hz), 4.32 (2H, q, J = 8 Hz), 4.99 (2H, s), 7.10 (1H, d, J = 12 Hz), 8.81 (1H, d, J = 8 Hz).
IR Nujol ($cm^{-1}$): 1735 (ester), 1580 (benzene ring), 1330 (nitro).
EI-MS m/z: 288 ($M^+$), 242, 214, 169.
(8) A suspension of ethyl 2,4-dinitro-5-chlorophenoxyacetate (2.0 g) and potassium fluoride (0.76 g) in N,N-dimethylformamide (10 g) was stirred at a temperature of 122 °C for 1.5 hours. After cooling, the reaction mixture was diluted with ice water (200 ml) and extracted with ethyl acetate. The organic layer was washed with water and saturated sodium chloride solution in order, dried over anhydrous magnesium sulfate and concentrated to give ethyl 2,4-dinitro-5-fluorophenoxyacetate (1.4 g; yield, 74.0 %).

Example 3

Production of the fluoroaniline derivative (I):-
(1) A suspension of 2,4-dinitro-5-fluorophenoxyacetic acid (0.25 g) and platinum dioxide (80 mg) in ethanol (10 ml) was subjected to catalytic reduction at room temperature under an ordinary pressure until 130 ml of hydrogen was absorbed. The reaction mixture was filtered, and the filtrate was concentrated to give 7-fluoro-6-amino-2H-1,4-benzoxazin-3(4H)-one (0.16 g; yield, 91.4 %). M.P., more than 300 °C.
NMR δ ($CDCl_3$-DMSO-$d_6$): 10.25 (1H), 6.70 (1H, d, J = 12 Hz), 6.54 (1H, d, J = 9 Hz), 4.50 (2H, s), 4.00 (2H).
IR Nujol ($cm^{-1}$): 3350 (NH), 1690 (CONH).
EI-MS m/z: 182 ($M^+$), 113.
(2) A suspension of 2,4-dinitro-5-fluorophenoxyacetic acid (2.0 g) and 10 % palladium-carbon (hydrated) (1.0 g) in ethanol (30 ml) was subjected to catalytic reduction at room temperature under an ordinary pressure until 1000 ml of hydrogen was absorbed. The reaction mixture was filtered, and the filtrate was concentrated to give 7-fluoro-6-amino-2H-1,4-benzoxazin-3(4H)-one (1.3 g; yield, 92.8 %).

(3) Iron powder (5.6 g) was suspended in 5 % acetic acid (112 g), and a solution of 2,4-dinitro-5-fluorophenoxyacetonitrile (2.4 g) in acetic acid (48 g) was dropwise added thereto under reflux, followed by refluxing for additional 1 hour. The reaction mixture was filtered, and the filtrate was extracted with ethyl acetate. The extract was concentrated, and the precipitated crystals were collected by filtration and washed with a mixture of hexane and ether to give 7-fluoro-6-amino-2H-1,4-benzoxazin-3(4H)-one (1.3 g; yield, 71.7 %).

(4) A suspension of 2,4-dinitro-5-fluorophenyl)glycine ethyl ester (2.0 g) and 10 % palladium-carbon (hydrated) (1.0 g) in ethanol (30 ml) was subjected to catalytic reduction at room temperature under an ordinary pressure until 1000 ml of hydrogen was absorbed. The reaction mixture was filtered, and the filtrate was concentrated to give 6-fluoro-7-amino-1,2-dihydroquinoxalin-2-one (1.24 g; yield, 99.4 %). M.P., more than 300 °C.

NMR δ ($CDCl_3$-DMSO-$d_6$): 10.00 (1H), 6.30 (1H), 6.42 (1H, d, J = 12 Hz), 6.39 (1H, d, J = 8 Hz), 4.02 (2H), 3.62 (2H, s).

IR Nujol ($cm^{-1}$): 3455 (NH), 1680 (CONH).

EI-MS m/z: 181 ($M^+$), 179, 151, 97.

(5) A suspension of ethyl 2,4-dinitro-5-fluorophenoxyacetate (1.3 g) and 5 % palladium-carbon (0.35 g) in ethanol (30 ml) was subjected to catalytic reduction at room temperature under an ordinary pressure until 600 ml of hydrogen was absorbed. The reaction mixture was filtered, and the filtrate was concentrated to give 7-fluoro-6-amino-2H-1,4-benzoxazin-3(4H)-one (0.68 g; yield, 82.8 %).

(6) N-(2,4-Dinitro-5-fluorophenyl)glycine ethyl ester (9.32 g), 2 % palladium-carbon (50 % hydrated) (10 g) and ethanol (200 ml) were charged into an autoclave, and catalytic reduction was carried out at room temperature under an initial pressure of 37 atm. After the reaction was completed, the reaction mixture was filtered, and the filtrate was concentrated to give 6-fluoro-7-amino-1,2-dihydroquinoxalin-2-one (4.0 g; yield, 68.0 %).

## Claims

**1.** A process for producing a fluoroaniline derivative of the formula:

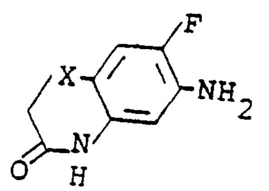

(I)

wherein X is an oxygen atom or an imino group, which comprises:

(a) reacting 1,5-dichloro-2,4-dinitrobenzene with a glycol derivative of the formula:

Y-$CH_2$-XH (III)

wherein X is as defined above and Y is a cyano group, a carboxy group or a lower alkoxycarbonyl group to produce a chlorodinitrobenzene derivative of the formula

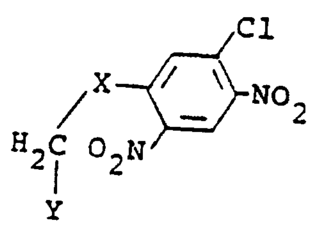

(IV)

wherein X and Y are each as defined above;

(b) reacting the chlorodinitrobenzene derivative (IV) with a metal fluoride to produce a fluorodinitrobenzene derivative of the formula:

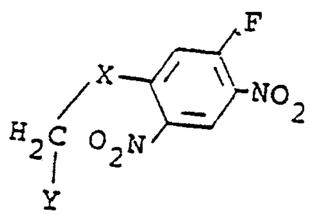

(II)

wherein X and Y are as defined above; and
(c) subjecting the fluorodinitrobenzene to reductive cyclization.

2. A process for producing a fluoroaniline derivative of the formula:

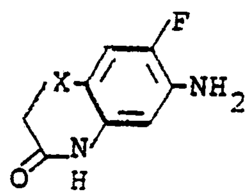

(I)

wherein X is an oxygen atom or an imino group, which comprises
(a) reacting 1,5-difluoro-2,4-dinitrobenzene with a glycol derivative of the formula:

$Y\text{-}CH_2\text{-}XH$ (III)

wherein X is as defined above and Y is a cyano group, a carboxy group or a lower alkoxycarbonyl group to produce a fluorodinitrobenzene derivative of the formula

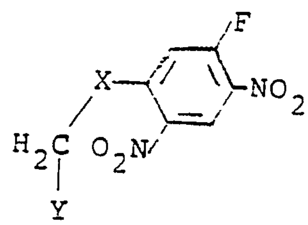

(II)

wherein X and Y are each as defined above, and
(b) subjecting the fluorodinitrobenzene to reductive cyclization.

3. The process according to claim 1, wherein step (c) is carried out in the presence of a catalyst under a hydrogen atmosphere.

4. The process according to claim 3, wherein the catalyst is platinum dioxide, palladium-carbon or Raney-nickel.

5. The process according to claim 1, wherein step (c) is carried out by chemical reduction.

6. The process according to claim 5, wherein the chemical reduction is performed in the presence of a metal and an acid.

7. The process according to claim 6, wherein the metal is iron powder or zinc powder.

**8.** The process according to claim 6, wherein the acid is hydrochloric acid, sulfuric acid or acetic acid.

**9.** The process according to claim 2, wherein the glycol derivative in step (a) is glycolonitrile, aminoacetonitrile, lower alkyl glycolate or glycine lower alkyl ester.

**10.** The process according to claim 2, wherein the molar ratio of 1,5-difluoro-2,4-dinitrobenzene and the glycol derivative in step (a) is 1 : 1 - 1.5.

**11.** The process according to claim 2, wherein the reaction in step (a) is carried out in the presence of a basic catalyst.

**12.** The process according to claim 11, wherein the basic catalyst is an inorganic base.

**13.** The process according to olaim 12, wherein the inorganic base is sodium hydroxide, potassium hydroxide, potassium carbonate or sodium hydrogen carbonate.

**14.** The process according to claim 11, wherein the basic catalyst is an organic amine.

**15.** The process according to claim 14, wherein the organic amine is pyridine, triethylamine or N,N-dimethylaniline.

**16.** The process according to claim 11, wherein the basic catalyst is used in an amount of 1 to 2 equivalents to one equivalent of 1,5-difluoro-2,4-dinitrobenzene.

**17.** The process according to claim 2, wherein the reaction in step (a) is effected at a temperature of -20 to 150 °C.

**18.** The process according to claim 1, wherein the metal fluoride in step (b) is an alkali metal fluoride or an alkaline earth metal fluoride.

**19.** The process according to claim 18, wherein the alkali metal fluoride is potassium fluoride.

**20.** The process according to claim 1, wherein the metal fluoride in step (b) is used in an amount of 1 to 5 equivalents to one equivalent of the chlorodinitrobenzene derivative.

**21.** The process according to claim 1, wherein step (b) is carried out in the presence of a phase transfer catalyst.

**22.** The process according to claim 21, wherein the phase transfer catalyst is used in an amount of 0.001 to 0.5 equivalents to one equivalent of the chlorodinitrobenzene derivative.

**23.** The process according to claim 1, wherein the glycol derivative in step (a) is glycolonitrile, aminoacetonitrile, lower alkyl glycolate or glycine lower alkyl ester.

**24.** The process according to claim 1, wherein the molar ratio of 1,5-dichloro-2,4-dinitrobenzene and the glycol derivative in step (a) is 1 : 1 - 5.

**25.** The process according to claim 1, wherein step (a) is carried out in the presence of a basic catalyst.

**26.** The process according to claim 25, wherein the basic catalyst is an inorganic base.

**27.** The process according to claim 26, wherein the inorganic base is sodium hydroxide, potassium hydroxide, potassium carbonate or sodium hydrogen carbonate.

**28.** The process according to claim 25, wherein the basic catalyst is an organic amine.

**29.** The process according to claim 28, wherein the organic amine is pyridine, triethylamine or N,N-dimethylaniline.

30. The process according to claim 25, wherein the basic catalyst is used in an amount of 1 to 2 equivalents to one equivalent of 1,5-dichloro-2,4-dinitrobenzene.

31. The process according to claim 1, wherein step (a) is carried out at a temperature of -20 to 150 ° C.

32. The process according to claim 2 wherein step (b) is carried out in the presence of a catalyst under a hydrogen atmosphere.

33. The process according to claim 32, wherein the catalyst is platinum dioxide, palladium carbon or Rancy nickel.

34. The process according to claim 2 wherein step (b) is carried out by chemical reduction.

35. The process according to claim 34, wherein the chemical reduction is performed in the presence of metal and an acid.

36. The process according to claim 35, wherein the metal is iron powder or zinc powder.

37. The process according to claim 35, wherein the acid is hydrochloric acid, sulfuric acid or acetic acid.

**Revendications**

1. Un procédé de production d'un dérivé de la fluoroaniline de formule :

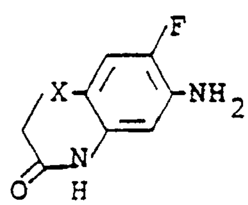

(I)

dans laquelle X est un atome d'oxygène ou un groupe imino, qui comprend :

(a) la réaction du 1,5-dichloro-2,4-dinitrobenzène avec un dérivé du glycol de formule :

$Y\text{-}CH_2\text{-}XH$ (III)

dans laquelle X est comme défini précédemment et Y est un groupe cyano, un groupe carboxy, un groupe alcoxycarbonyle inférieur pour produire un dérivé chlorodinitrobenzène de formule :

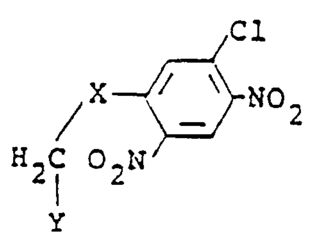

(IV)

dans laquelle X et Y sont chacun comme défini précédement ;

(b) la réaction du dérivé chlorodinitrobenzène (IV) avec un fluorure métallique pour produire un dérivé fluorodinitrobenzène de formule :

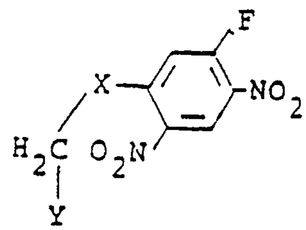

(II)

dans laquelle X et Y sont comme défini ci-dessus ; et
(c) le traitement du fluorodinitrobenzène par cyclisation réductrice.

2. Un procédé de production d'un dérivé de la fluoroaniline de formule :

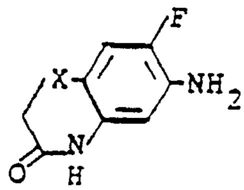

(I)

dans laquelle X est un atome d'oxygène ou un groupe imino, qui comprend :
(a) la réaction du 1,5-difluoro-2,4-dinitrobenzène avec un dérivé du glycol de formule :

$Y\text{-}CH_2\text{-}XH$ (III)

dans laquelle X est comme défini précédemment et Y est un groupe cyano, un groupe carboxy ou un groupe alcoxycarbonyle inférieur pour produire un dérivé fluorodinitrobenzène de formule :

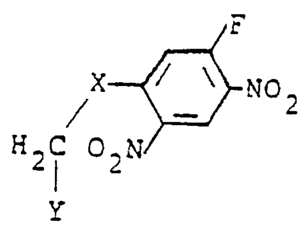

(II)

dans laquelle X et Y sont chacun comme défini précédemment ; et
(b) le traitement du fluorodinitrobenzène par cyclisation réductrice.

3. Le procédé selon la revendication 1, selon lequel l'étape (c) est conduite en présence d'un catalyseur sous une atmosphère d'hydrogène.

4. Le procédé selon la revendication 3, selon lequel le catalyseur est le dioxyde de platine, le palladium sur support de charbon ou le nickel Raney.

5. Le procédé selon la revendication 1, selon lequel l'étape (c) est conduite par réduction chimique.

6. Le procédé selon la revendication 5, selon lequel la réduction chimique est conduite en présence d'un métal et d'un acide.

7. Le procédé selon la revendication 6, selon lequel le métal est la poudre de fer ou la poudre de zinc.

8. Le procédé selon la revendication 6, selon lequel l'acide est l'acide chlorhydrique, l'acide sulfurique ou l'acide acétique.

9. Le procédé selon la revendication 2, selon lequel le dérivé du glycol dans l'étape (a) est le glycolonitrile, l'aminoacétonitrile, un glycolate d'alkyle inférieur ou un ester d'alkyle inférieur de la glycine.

10. Le procédé selon la revendication 2, selon lequel le rapport molaire entre le 1,5-difluoro-2,4-dinitrobenzène et le dérivé du glycol dans l'étape (a) est de 1:1-1,5.

11. Le procédé selon la revendication 2, selon lequel la réaction dans l'étape (a) est conduite en présence d'un catalyseur basique.

12. Le procédé selon la revendication 11, selon lequel le catalyseur basique est une base inorganique.

13. Le procédé selon la revendication 12, selon lequel la base organique est l'hydroxyde de sodium, l'hydroxyde de potassium, le carbonate de potassium, ou l'hydrogénocarbonate de sodium.

14. Le procédé selon la revendication 11, selon lequel le catalyseur basique est une amine organique.

15. Le procédé selon la revendication 14, selon lequel l'amine organique est la pyridine, la triéthylamine ou la N,N-diméthylaniline.

16. Le procédé selon la revendication 11, selon lequel le catalyseur basique est utilisé en quantité de 1 à 2 équivalents par équivalent de 1,5-difluoro-2,4-dinitrobenzène.

17. Le procédé selon la revendication 2, selon lequel la réaction dans l'étape (a) est effectuée à une température de -20 à 150 °C.

18. Le procédé selon la revendication 1, selon lequel le fluorure métallique dans l'étape (b) est un fluorure de métal alcalin ou un fluorure de métal alcalino-terreux.

19. Le procédé selon la revendication 18, selon lequel le fluorure de métal alcalin est le fluorure de potassium.

20. Le procédé selon la revendication 1, selon lequel le fluorure métallique dans l'étape (b) est utilisé en quantité de 1 à 5 équivalents par équivalent de dérivé chlorodinitrobenzène.

21. Le procédé selon la revendication 1, selon lequel l'étape (b) est conduite en présence d'un catalyseur de transfert de phase.

22. Le procédé selon la revendication 21, selon lequel le catalyseur de transfert de phase est utilisé en quantité de 0,001 à 0,5 équivalent par équivalent de dérivé chlorodinitrobenzène.

23. Le procédé selon la revendication 1, selon lequel le dérivé du glycol dans l'étape (a) est le glycolonitrile, l'aminoacétonitrile, un glycolate d'alkyle inférieur ou un ester d'alkyle inférieur de la glycine.

24. Le procédé selon la revendication 1, selon lequel le rapport molaire entre le 1,5-dichloro-2,4-dinitrobenzène et le dérivé du glycol dans l'étape (a) est de 1:1-5.

25. Le procédé selon la revendication 1, selon lequel l'étape (a) est conduite en présence d'un catalyseur basique.

26. Le procédé selon la revendication 25, selon lequel le catalyseur basique est une base inorganique.

27. Le procédé selon la revendication 26, selon lequel la base inorganique est l'hydroxyde de sodium, l'hydroxyde de potassium, le carbonate de potassium ou l'hydrogénocarbonate de sodium.

**28.** Le procédé selon la revendication 25, selon lequel le catalyseur basique est une amine organique.

**29.** Le procédé selon la revendication 28, selon lequel l'amine organique est la pyridine, la triéthylamine ou la N,N-diméthylaniline.

**30.** Le procédé selon la revendication 25, selon lequel le catalyseur basique est utilisé en quantité de 1 à 2 équivalents par équivalent de 1,5-dichloro-2,4-dinitrobenzène.

**31.** Le procédé selon la revendication 1, selon lequel l'étape (a) est conduite à une température de -20 à 150 °C.

**32.** Le procédé selon la revendication 2, selon lequel l'étape (b) est conduite en présence d'un catalyseur sous une atmosphère d'hydrogène.

**33.** Le procédé selon la revendication 32, selon lequel le catalyseur est le dioxyde de platine, le palladium sur support de charbon ou le nickel de Raney.

**34.** Le procédé selon la revendication 2, selon lequel l'étape (b) est conduite par réduction chimique.

**35.** Le procédé selon la revendication 34, selon lequel la réaction chimique est conduite en présence d'un métal et d'un acide.

**36.** Le procédé selon la revendication 35, selon lequel le métal est la poudre de fer ou la poudre de zinc.

**37.** Le procédé selon la revendication 35, selon lequel l'acide est l'acide chlorhydrique, l'acide sulfurique ou l'acide acétique.

**Patentansprüche**

**1.** Verfahren zur Herstellung eines Fluoranilin-Derivats der Formel:

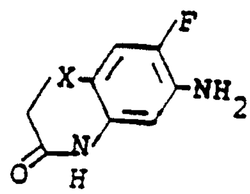

(I)

in der X ein Sauerstoffatom oder eine Iminogruppe bedeutet, umfassend:

(a) Umsetzung von 1,5-Dichlor-2,4-dinitrobenzol mit einem Glykolderivat der Formel:

$Y\text{-}CH_2\text{-}XH$ (III)

in der X die vorstehend angegebene Bedeutung hat und Y eine Cyanogruppe, eine Carboxygruppe oder einen Niederalkoxycarbonylrest darstellt, zur Herstellung eines Chlordinitrobenzol-Derivats der Formel:

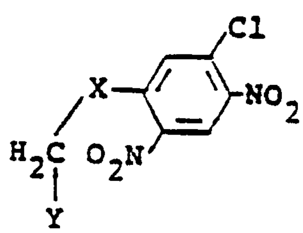

(IV)

in der X und Y jeweils die vorstehend angegebene Bedeutung haben;
(b) Umsetzung des Chlordinitrobenzol-Derivats (IV) mit einem Metallfluorid zur Herstellung eines Fluordinitrobenzol-Derivats der Formel:

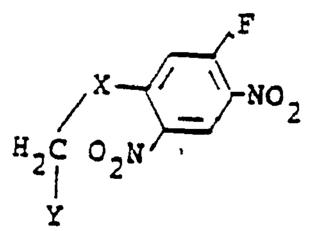

(II)

in der X und Y die vorstehend angegebene Bedeutung haben; und
(c) reduktive Cyclisierung des Fluordinitrobenzols.

2. Verfahren zur Herstellung eines Fluoranilin-Derivats der Formel:

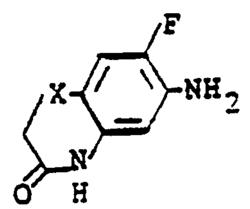

(I)

in der X ein Sauerstoffatom oder eine Iminogruppe darstellt, umfassend:
(a) Umsetzung von 1,5-Difluor-2,4-dinitrobenzol mit einem Glykolderivat der Formel:

$Y-CH_2-XH$ (III)

in der X die vorstehend angegebene Bedeutung hat und Y eine Cyanogruppe, eine Carboxygruppe oder einen Niederalkoxycarbonylrest bedeutet, zur Herstellung eines Fluordinitrobenzol-Derivats der Formel

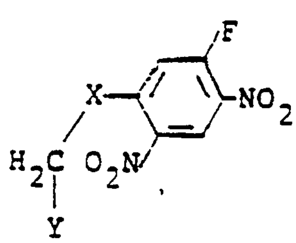

(II)

in der X und Y jeweils die vorstehend angegebene Bedeutung haben, und
(b) reduktive Cyclisierung des Fluordinitrobenzols.

3. Verfahren nach Anspruch 1, wobei Schritt (c) in Gegenwart eines Katalysators in einer Wasserstoffatmosphäre durchgeführt wird.

4. Verfahren nach Anspruch 3, wobei der Katalysator Platindioxid, Palladium-Kohlenstoff oder Raney-Nickel ist.

5. Verfahren nach Anspruch 1, wobei Schritt (c) als chemische Reduktion ausgeführt wird.

6. Verfahren nach Anspruch 5, wobei die chemische Reduktion in Gegenwart eines Metalls und einer Säure durchgeführt wird.

7. Verfahren nach Anspruch 6, wobei das Metall Eisen- oder Zinkpulver ist.

8. Verfahren nach Anspruch 6, wobei die Säure Salzsäure, Schwefelsäure oder Essigsäure ist.

9. Verfahren nach Anspruch 2, wobei das Glykolderivat in Schritt (a) Glykolnitril, Aminoacetonitril, Niederalkylglykolat oder ein Glycinniederalkylester ist.

10. Verfahren nach Anspruch 2, wobei das Molverhältnis von 1,5-Difluor-2,4-dinitrobenzol zu dem Glykolderivat in Schritt (a) 1 : 1 - 1,5 beträgt.

11. Verfahren nach Anspruch 2, wobei die Umsetzung in Schritt (a) in Gegenwart eines basischen Katalysators durchgeführt wird.

12. Verfahren nach Anspruch 11, wobei der basische Katalysator eine anorganische Base ist.

13. Verfahren nach Anspruch 12, wobei die anorganische Base Natriumhydroxid, Kaliumhydroxid, Kaliumcarbonat oder Natriumhydrogencarbonat ist.

14. Verfahren nach Anspruch 11, wobei der basische Katalysator ein organisches Amin ist.

15. Verfahren nach Anspruch 14, wobei das organische Amin Pyridin, Triethylamin oder N,N-Dimethylanilin ist.

16. Verfahren nach Anspruch 11, wobei der basische Katalysator in einer Menge von 1 bis 2 Äquivalenten zu einem Äquivalent des 1,5-Difluor-2,4-dinitrobenzols eingesetzt wird.

17. Verfahren nach Anspruch 2, wobei die Umsetzung in Schritt (a) bei einer Temperatur von -20 bis 150 °C ausgeführt wird.

18. Verfahren nach Anspruch 1, wobei das Metallfluorid in Schritt (b) ein Alkalimetallfluorid oder ein Erdalkalimetallfluorid ist.

19. Verfahren nach Anspruch 18, wobei das Alkalimetallfluorid Kaliumfluorid ist.

20. Verfahren nach Anspruch 1, wobei das Metallfluorid in Schritt (b) in einer Menge von 1 bis 5 Äquivalenten zu einem Äquivalent des Chlordinitrobenzol-Derivats verwendet wird.

21. Verfahren nach Anspruch 1, wobei Schritt (b) in Gegenwart eines Phasentransfer-Katalysators durchgeführt wird.

22. Verfahren nach Anspruch 21, wobei der Phasentransfer-Katalysator in einer Menge von 0,001 bis 0,5 Äquivalenten zu einem Äquivalent des Chlordinitrobenzol-Derivats verwendet wird.

23. Verfahren nach Anspruch 1, wobei das Glykolderivat in Schritt (a) Glykolnitril, Aminoacetonitril, Niederalkylglykolat oder ein Glycinniederalkylester ist.

24. Verfahren nach Anspruch 1, wobei das Molverhältnis von 1,5-Dichlor-2,4-dinitrobenzol zu dem Glykolderivat in Schritt (a) 1 : 1 - 5 beträgt.

25. Verfahren nach Anspruch 1, wobei Schritt (a) in Gegenwart eines basischen Katalysators durchgeführt wird.

26. Verfahren nach Anspruch 25, wobei der basische Katalysator eine anorganische Base ist.

27. Verfahren nach Anspruch 26, wobei die anorganische Base Natriumhydroxid, Kaliumhydroxid, Kaliumcarbonat oder Natriumhydrogencarbonat ist.

28. Verfahren nach Anspruch 25, wobei der basische Katalysator ein organisches Amin ist.

29. Verfahren nach Anspruch 28, wobei das organische Amin Pyridin, Triethylamin oder N,N-Dimethylanilin ist.

30. Verfahren nach Anspruch 25, wobei der basische Katalysator in einer Menge von 1 bis 2 Äquivalenten zu einem Äquivalent des 1,5-Dichlor-2,4-dinitrobenzols verwendet wird.

31. Verfahren nach Anspruch 1, wobei Schritt (a) bei einer Temperatur von -20 bis 150 °C durchgeführt wird.

32. Verfahren nach Anspruch 2, wobei Schritt (b) in Gegenwart eines Katalysators in einer Wasserstoffatmosphäre durchgeführt wird.

33. Verfahren nach Anspruch 32, wobei der Katalysator Platindioxid, Palladium-Kohlenstoff oder Raney-Nickel ist.

34. Verfahren nach Anspruch 2, wobei Schritt (b) als chemische Reduktion ausgeführt wird.

35. Verfahren nach Anspruch 34, wobei die chemische Reduktion in Gegenwart eines Metalls und einer Säure durchgeführt wird.

36. Verfahren nach Anspruch 35, wobei das Metall Eisen- oder Zinkpulver ist.

37. Verfahren nach Anspruch 35, wobei die Säure Salzsäure, Schwefelsäure oder Essigsäure ist.